# EUROPÄISCHE PATENTSCHRIFT

(11) **EP 0 324 490 B1**
(45) Veröffentlichungstag und Bekanntmachung des Hinweises auf die Patenterteilung: **25.08.1993**
(21) Anmeldenummer: 89100512.6
(22) Anmeldetag: 13.01.1989
(51) Int. Cl.: A61N 5/06

(54) **Vorrichtung zur grossflächigen Bestrahlung**
Large surface irradiation device
Dispositif d'irradiation à grande surface

(30) Priorität: 15.01.1988 DE 3801027
(43) Veröffentlichungstag der Anmeldung: 19.07.1989
(73) Patentinhaber: SAUNALUX GMBH PRODUCTS & CO KG, 36355 Grebenhain (DE)
(72) Erfinder: Schneider, Heinz, D-7311 Erkenbrechtsweiler (DE)
(74) Vertreter: Linser, Heinz

(56) Entgegenhaltungen:
- DE-A- 2 937 598
- DE-A- 2 943 117
- DE-A- 3 600 464

## Beschreibung

Die Erfindung betrifft eine Vorrichtung zur großflächigen Bestrahlung des menschlichen Körpers oder seiner Teile, mit einer Anordnung von verschiedenen Lampen und/Lampengruppen, welche IR-Strahlung, Licht und UV-Strahlung aussenden, mit Mitteln zur gerichteten Aussendung und Filterung der Strahlung und einem programmierbaren Rechner.

Zur therapeutischen und kosmetischen Behandlung insbesondere der Haut sind die verschiedensten Lampen und Strahler bekannt, welche jeweils ein spezifisches vom Lampentyp abhängiges Strahlungsspektrum aussenden. Jedes Spektrum zeigt dabei unterschiedliche Wirkungen auf die Haut. Für fast alle biologischen Bestrahlungswirkungen ist die fotobiologische Bestrahlung maßgebend, worunter das Produkt aus fotobiologisch wirksamer Bestrahlungsstärke und Zeit verstanden wird.

Zur Kennzeichnung einer bestimmten Wirkung in Abhängigkeit von der Wellenlänge der Strahlung sind entsprechende Wirkungsfunktionen eingeführt. Hierzu wird auf die DIN 5031 Teil 10 verwiesen.

Es sind bereits Bestrahlungsgeräte bekannt, welche mit mehreren Lampen unterschiedlicher Spektralverteilung ausgerüstet sind, beispielsweise IR-Strahler, Leuchtstofflampen, Hg-Hochdrucklampen oder Kombinationen daraus. Jede Lampe oder Gruppe von Lampen läßt sich mit einer Zeituhr einschalten und zur Begrenzung der Bestrahlungsdauer steuern, so daß auch verschiedene Strahlungsbereiche mit unterschiedlichen Bestrahlungszeiten eingesetzt werden können. Mit derartigen Strahlungsgeräten ist weder eine genaue noch eine automatische Bestimmung der Dosis, d.h. dem Körper zugeführte Bestrahlungsmenge möglich, da bereits der momentane bestrahlungstechnische Zustand der Lampen nicht bekannt ist oder berücksichtigt werden kann.

Desgleichen läßt sich eine Vorbestimmung der zu verabreichenden Strahlendosis nicht festlegen, da auch die individuellen Faktoren der zu bestrahlenden Personen nicht berücksichtigt werden, welche für die individuelle Empfindlichkeit maßgebend sind, sondern höchstens in einer groben Abschätzung bei der Festlegung der Gesamtbestrahlungszeit einbezogen werden. Eine solche Vorrichtung geht beispielsweise aus der DE 29 37 598 hervor. Dieser Literaturstelle liegt die Aufgabe zugrunde, ein Verfahren und eine Vorrichtung zur vorprogrammierbaren Bestrahlung anzugeben, mit dessen Hilfe mehrere Variablen berücksichtigt und im Laufe einer Behandlung korrigiert werden können. Die Lösung dieses Problems erfolgt mit Hilfe eines Programmträgers, wie einer Magnetkarte oder dergleichen, der persönliche Behandlungsdaten enthält, aus denen von einem Rechner die erforderlichen Daten für die Bestrahlungszeit und die spektrale Emission ermittelt und am Bestrahlungsgerät eingestellt werden. Diese lassen sich jedoch nach der Einstellung nicht mehr verändern oder anpassen und entsprechend ist es nicht möglich unterschiedliche Spektralbereiche zu unterschiedlichen Zeiten in einem Bestrahlungsgang zum Einsatz zu bringen.

Der Erfindung liegt somit die Aufgabe zugrunde eine Vorrichtung anzugeben, mit der unter Berücksichtigung der noch zu ermittelnden individuellen Strahlungsempfindlichkeit einer zu bestrahlenden Person und des momentanen strahlungstechnischen Zustandes des Bestrahlungsgerätes eine Dosis unter Berücksichtigung unterschiedlicher Strahlungsbereiche vorbestimmbar ist und durch Überprüfung, Messung und automatischer Steuerung des Gerätes während der Bestrahlung genau einzuhalten ist.

Die Lösung dieser Aufgabe erfolgt nach der Erfindung bei der eingangs genannten Vorrichtung dadurch, daß die Lampen und/oder Lampengruppen von einer gemeinsamen rechnergesteuerten Steuervorrichtung zur Erzeugung kombinierter unterschiedlicher Spektren, Strahlungszeiten und Intensitäten während der Gesamtbestrahlungszeit in einem Bereich der optischen Strahlung beaufschlagbar und steuerbar ausgebildet sind, wobei jede Lampe oder Lampengruppe einen Sensor zur ständigen oder intermittierenden Messung der ausgehenden Strahlungsleistung der zugeordneten Lampe oder Lampengruppe aufweist, der mit der Steuervorrichtung elektrisch verbunden ist und dessen Ausgangssignale dem Rechner digitalisiert zuführbar sind und der mit personenspezifischen Daten beaufschlagbare Rechner derartig ausgebildet ist, daß er aus diesen und den gemessenen lampenspezifischen Größen für jede Lampe oder Lampengruppe Steuergrößen ermittelt, welche zur Beaufschlagung der Steuervorrichtung und Aussendung einer personenbezogenen Strahlendosis dienen.

Zur Ermittlung der individuellen fotobiologischen Wirkung der Bestrahlung durch die einzelnen Lampen und/oder Lampengruppen werden die persönlichen Merkmale der zu bestrahlenden Person, wie Geschlecht, Alter, Hauttyp und Anzahl der vorangegangenen Bestrahlungen sowie die Jahreszeit erfaßt und in Kennwerte gewandelt dem Rechner der Steuereinrichtung eingegeben. Zusammenhänge zwischen diesen Merkmalen und der Bestrahlungsdosis sind bekannt. Hierzu wird auf die bereits genannte DIN 5031 verwiesen. Aus diesen so ermittelten Kennwerten läßt sich die zulässige Bestrahlung als Soll-Wert, bezogen auf die einzelnen Lampen oder Lampengruppen errechnen und als Steuergrößen im Rechner speichern.

Auf dieser Basis läßt sich daher ein individueller Bestrahlungsplan erstellen, welcher dem Bestrahlungsgerät als Ablaufplan zur Steuerung der einzelnen Bestrahlungslampen vorliegt.

Je nach der zu behandelnden Erkrankung und der entsprechenden Körperteile wird somit der benötigte Spektalbereich oder verschiedene Bereiche entsprechend der vorhandenen Lampen und deren Einschalt-und Ausschaltzeit, d.h. ihre jeweilige Bestrahlungszeit in Relation untereinander im voraus festgelegt und erst nach Eingabe der individuellen Merkmale bzw. Kennwerte und Berechnung des Soll-Wertes für die einzelnen Lampen bzw. Lampengrupen wird das Bestrahlungsgerät zur Gesamtbestrahlung freigegeben.

Der Rechner errechnet in einer vorteilhaften Ausführungsform der Erfindung ferner nach Durchführung einer Bestrahlung den Zeitpunkt einer folgenden Bestrahlung, bezogen auf die bestrahlte Person, und gibt diesen Wert auf einer Anzeigevorrichtung, beispielsweise einem Bildschirm aus.

Nach Durchführung einer Bestrahlung ist es mit Hilfe eines dem Rechner angeschlossenen Druckers auch vorteilhaft, ein Protokoll über die durchgeführte Bestrahlung auszudrucken, so daß über den gesamten Bestrahlungsvorgang dokumentarische Unterlagen automatisch angefertigt werden.

Die Lampen und/oder Lampengrupen sind von einer gemeinsamen mit einem programmierbaren Rechner kombinierten Steuervorrichtung zur Erzeugung kombinierter unterschiedlicher Spektren während der Bestrahlungszeit in einem Wellenlängenbereich von etwa 230 bis 3000 nm beaufschlagbar und steuerbar ausgebildet.

Nach der Erfindung ist jede Lampe oder Lampengruppe, wie eingangs aufgeführt, mit einem Sensor zur ständigen oder intermittierenden Messung der Strahlungsleistung der zugeordneten Lampe oder Lampengruppe versehen. Der Sensor ist dabei mit der Steuereinrichtung elektrisch verbunden und dessen Ausgangssignale sind digitalisiert dem Rechner zuführbar.

Durch diese Maßnahme nach der Erfindung wird in objektiver Weise der jeweils momentane Zustand des Bestrahlungsgerätes in die Rechenoperationen mit einbezogen, so daß die verabreichte Dosis fortlaufend bestimmbar ist und den sich ändernden Gegebenheiten anpassen läßt.

Der mit der Steuervorrichtung nach der Erfingung kombinierte programmierbare Rechner errechnet für jede Lampe oder Lampengruppe die eingegebenen Daten zu einem (Dosis) Soll-Wert und die von den Sensoren aufgenommenen Werte für jede Lampe oder Lampengruppe zu einem Ist-Wert und verarbeitet diese zur Erzeugung von Steuerbefehlen, wobei die Differenz der Soll-Ist-Werte die Führungsgröße darstellt.

Die Steuereinrichtung für jede zu steuernde Lampe oder Lampengruppe weist zwei parallel geschaltete Additionsglieder auf, welche mit einer gemeinsamen Basisgröße beaufschlagbar sind, wobei einem Additionsglied gerätespezifische Einflußgrößen zuführbar sind, welche über ein Verzögerungsglied einem Komparator als Ist-Wert zuleitbar sind. Das zweite Additionsglied im Parallelkreis wird mit den personenspezifischen Einflußgrößen beaufschlagt, welche dem zweiten Eingang des Komparators als Soll-Wert zugeführt werden, wobei die Differenz der Komparatoreingangswerte die Führungsgröße bildet.

Dem mit der Steuervorrichtung kombinierten Rechner ist ein A/D-Wandler und diesem ein Glied zur Abfrage einer großen Zahl von Eingangsgrößen vorgeschaltet und ein D/A-Wandler nachgeschaltet. Der D/A-Wandler weist ein Ausgangsglied zum Ansteuern vieler mit Adresse versehenen Schaltglieder auf.

Die Erfindung wird anhand der Zeichnungen näher erläutert.

Hierbei zeigen:
- FIGUR 1: eine Grundschaltung zur Ermittlung der Führungs- und Stellgrößen für die einzelnen Lampen oder Lampengruppen, und
- FIGUR 2: den Aufbau einer Steuereinrichtung unter Verwendung eines Rechners.

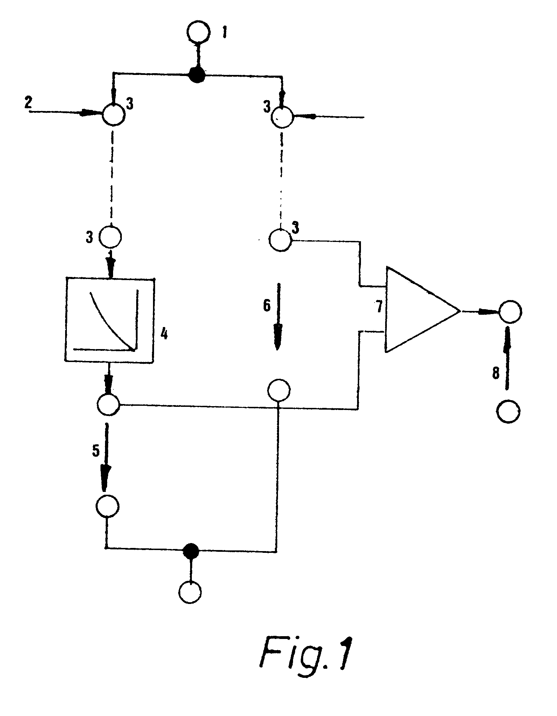

Nach Figur 1 weist die Steuereinrichtung für jede zu steuernde und nicht näher dargestellte Lampe oder Lampengruppe zwei parallel geschaltete Additionsglieder 3, 3′ auf, welche mit einer gemeinsamen Basisgröße 1 beaufschlagt werden. Dem Additionsglied 3 werden gerätespezifische Einflußgrößen zugeführt, welche über ein Verzögerungsglied 4 einem Komparator 7 als Ist-Wert eingespeist werden. Das zweite Additionsglied 3′, welches parallel zum Additionsglied 3 liegt, wird mit den personenspezifischen Einflußgrößen beaufschlagt, welche dem zweiten Eingang des Komparators 7 als Soll-Wert zugeführt werden, wobei die Differenz der Komparatoreingangswerte die Führungsgröße 6 bildet. Der Pfeil 8 zeigt die Stellgröße an. Die Verkopplung der Kreise erfolgt somit mit Hilfe von einstellbaren Additionsgliedern 3 und 3′.

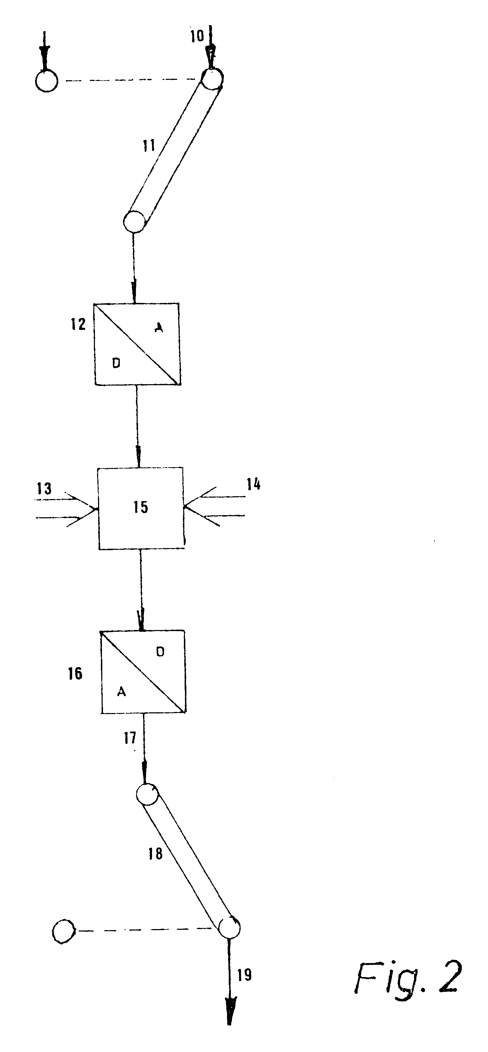

Die Figur 2 zeigt eine Schaltung, bei der ein Programm das Errechnen der Stellgrößen aus den Eingangsgrößen unter Verwendung eines Rechners 15 durchführt. Die Vielseitigkeit eines programmgesteuerten Rechners erlaubt dabei beliebige Verkopplungen.

Werden z.B. nur die wichtigten personenspezifischen Einflußgrößen wie Geschlecht, Hauttyp, Alter und Anzahl der vorangegangenen Bestrahlungen sowie die Jahreszeit mit der tatsächlichen Bestrahlungsstärke verkoppelt, so ergeben sich für jede Lampengruppe 128 Variationen und bei drei Lampengruppen bereits 768 Bestrahlungsvariationen. Eine solche Steuervorrichtung zeigt die Figur 2 in schematischer Darstellung.

Hierbei zeigen 10 die n Regelgrößen, welche einem Analog-Digital-Wandler 12 über ein entsprechendes Abfrageglied 11 zugeführt werden, der mit dem Rechner 15 verbunden ist. Dieser wird mit den Führungsgrößen 13 einerseits und dem Regelalgorithmus 14 andererseits beaufschlagt. Die von dem Rechner 15 errechneten Größen werden über einen Digital-Analog-Wandler 16 als Stellgrößen 17 abgegeben, mit denen Ausgangsglieder 18 zur Ansteuerung einer größeren Zahl von Schaltgliedern 19 mit Adresse als Befehlssignal gespeist werden.

Das Gerät nach der Erfindung erlaubt es nunmehr eine Vielzahl von individuellen Daten einer zu bestrahlenden Person zur Ermittlung einer beabsichtigten Strahlendosis zu verwenden um diese zu errechnen, wobei gerätespezifische Daten zur Feststellung der tatsächlich eingesetzten Dosis gemessen und während des Bestrahlungsvorganges berücksichtigt werden.

Die Erstellung eines dokumentarischen Protokolls sowohl der eingegebenen als auch der gemessenen gerätespezifisachen Daten über den zeitlichen Ablauf der Bestrahlung mit einer unterschiedlichen Art von Lampen und Lampengruppen, ermöglicht eine zusätzliche Kontrolle und Überwachung des Geschehens, welches nicht nur der Sicherheit zu bestrahlender Personen dient, sondern auch für Forschungszwecke in vielfacher Weise verwendet werden kann.

## Patentansprüche

1. Vorrichtung zur großflächigen Bestrahlung des menschlichen Körpers oder seiner Teile, mit einer Anordnung von verschiedenen Lampen und/Lampengruppen, welche IR-Strahlung, Licht und UV-Strahlung aussenden, mit Mitteln zur gerichteten Aussendung und Filterung der Strahlung und einem programmierbaren Rechner, wobei die Lampen und/oder Lampengruppen von einer gemeinsamen rechnergesteuerten Steuervorrichtung zur Erzeugung kombinierter unterschiedlicher Spektren, Strahlungszeiten und Intensitäten während der Gesamtbestrahlungszeit in einem Bereich der optischen Strahlung beaufschlagbar und steuerbar ausgebildet sind, und jede Lampe oder Lampengruppe einen Sensor zur ständigen oder intermittierenden Messung der ausgehenden Strahlungsleistung der zugeordneten Lampe oder Lampengruppe aufweist, der mit der Steuervorrichtung elektrisch verbunden ist und dessen Ausgangssignale dem Rechner digitalisiert zuführbar sind und der mit personenspezifischen Daten beaufschlagbare Rechner derartig ausgebildet ist, daß er aus diesen und den gemessenen lampenspezifischen Größen für jede Lampe oder Lampengruppe Steuergrößen ermittelt, welche zur Beaufschlagung der Steuervorrichtung und Aussendung einer personenbezogenen Strahlendosis dienen und der mit der Steuervorrichtung kombinierte Rechner die eingegebenen Daten zu einem Soll-Wert für jede Lampe oder Lampengruppe errechnet und die von den Sensoren aufgenommenen Werte zu einem Ist-Wert für jede Lampe oder Lampengruppe zur Erzeugung von Steuerbefehlen verarbeitet, wobei die Differenz der Soll-Ist-Werte die Führungsgröße darstellt.

2. Vorrichtung nach Anspruch 1, **dadurch gekennzeichnet, daß** die Steuervorrichtung für jede zu steuernde Lampe oder Lampengruppe zwei parallel geschaltete Additionsglieder aufweist, welche mit einer gemeinsamen Basisgröße beaufschlagbar sind, wobei einem Additionsglied gerätespezifische Einflußgrößen zuführbar sind, welche über ein Verzögerungsglied einem Komparator als Ist-Wert zuleitbar sind und das zweite Additionsglied im Parallelkreis mit den personenspezifischen Einflußgrößen beaufgschlagbar ist, welche dem zweiten Eingang des Komparators als Soll-Wert zugeführt werden, wobei die Differenz der Komparatoreingangswerte die Führungsgröße bildet.

3. Vorrichtung nach Anspruch 1 oder 2, **dadurch gekennzeichnet, daß** dem mit der Steuervorrichtung kombinierten Rechner ein A/D-Wandler und diesem ein Glied zur Abfrage einer großen Zahl von Eingangsgrößen vorgeschaltet ist und ein D/A-Wandler nachgeschaltet ist und der D/A-Wandler ein Ausgangsglied zum Ansteuern vieler mit Adresse versehenen Schaltglieder aufweist.

4. Vorrichtung nach Anspruch 1 bis 3, **dadurch gekennzeichnet, daß** die Lampen und/oder Lampengrupen von einer gemeinsamen mit einem programmierbaren Rechner kombinierten Steuervorrichtung zur Erzeugung kombinierter unterschiedlicher Spektren während der Bestrahlungszeit in einem Wellenlängenbereich von etwa 230 bis 3000 nm beaufschlagbar und steuerbar ausgebildet sind.

## Claims

1. Apparatus for extensive radiation therapy of the human body or its parts, with an arrangement of different lamps and/or groups of lamps which emit IR-radiation, light radiation, and UV-radiation, with means for directing the emitted radiation and for filtering the radiation and with a programmable computer, wherein the lamps and/or groups of lamps are formed into a combination under the command of a common computer-controlled system-controller for producing combined and differing spectra, radiation periods, and intensities during the total radiation period of one optical radiation zone; and each lamp or group of lamps has a sensor for constant or intermittent measurement of the radiation output from its respective lamp or group of lamps, which sensor is electrically connected to the system-controller, and the output signals of which can be digitalized and fed to the computer; and the computer, which can be supplied with person-specific data, is arranged such that from these data, and from the measured, lamp-specific quantities, it determines for each lamp or group of lamps control quantities which serve to command the system-controller and to emit a person-related radiation dose; and the computer together with the system-controller calculates from the input data a theoretical value for each lamp or group of lamps, and processes the values from the sensors into an actual value for each lamp or group of lamps, thus generating control commands whereof the difference in the theoretical and actual values represents the operating quantity.

2. Apparatus according to Claim 1, characterised in that the system-controller has, for each lamp or group of lamps to be controlled, two summing devices connected in parallel and supplied with a common reference quantity, whereof one summing device is fed with instrument-specific input quantities which are supplied via a delay device to a comparator as an actual value, and the second summing device in the parallel circuit is supplied with the person-specific input quantities, which are fed to the second input of the comparator as a theoretical value, whereby the difference in the comparator input values constitutes the operating quantity.

3. Apparatus according to Claim 1 or 2, characterised in that an A/D-converter is connected upstream of the computer/system-controller combination, to which converter is in turn connected a component for examining a large number of input quantities, and a downstream-connected D/A-converter has an output device for controlling many circuit components with different addresses.

4. Apparatus according to Claims 1 to 3, characterised in that the lamps and/or groups of lamps, together with the programmable computer, form an overall controllable assembly for producing combined and differing spectra during the radiation period in a wave-length band of approximately 230 to 3000 nm.

## Revendications

1. Dispositif pour irradier une grande surface du corps humain ou d'une partie de celui-ci, avec un agencement de diverses lampes et/ou groupes de lampes émettant un rayonnement IR, lumineux et UV, avec des moyens pour l'émission dirigée et le filtrage du rayonnement et un calculateur programmable, dans lequel les lampes et/ou groupes de lampes sont réalisés de manière à être alimentés et commandés par un dispositif de commande commun contrôlé par calculateur, pour produire des temps de rayonnement, intensités et spectres différents combinés pendant la durée globale du rayonnement dans un domaine du rayonnement optique, et chaque lampe ou groupe de lampes présente un détecteur pour mesurer de manière permanente ou intermittente la puissance de rayonnement émise par la lampe ou le groupe de lampes associé, détecteur qui est relié électriquement au dispositif de commande et dont les signaux de sortie sont conduits au calculateur sous une forme numérique, et qui est réalisé avec un calculateur alimenté par des données spécifiques à la personne, de telle sorte qu'il détermine à partir de celles-ci et des grandeurs spécifiques des lampes, pour chaque lampe ou groupe de lampes, des grandeurs de commande qui servent à alimenter le dispositif de commande et à émettre une dose de rayonnement adaptée à la personne, et le calculateur combiné au dispositif de commande, calcule à partir des données introduites une valeur de consigne pour chaque lampe ou groupe de lampes, et traite les valeurs reçues des détecteurs en une valeur effective pour chaque lampe ou groupe de lampes, pour produire des instructions de commande, tandis que la différence entre la valeur de consigne et la valeur effective représente la grandeur de commande.

2. Dispositif selon la revendication 1, caractérisé en ce que le dispositif de commande présente pour chaque lampe ou groupe de lampes à commander, deux circuits d'addition relié en parallèle, alimentés par une grandeur de base commune, tandis qu'un circuit d'addition reçoit des grandeurs d'entrée spécifiques à l'appareil, qui sont conduites par un circuit d'amplification à un comparateur sous la forme de valeur effective, et le second circuit d'addition dans le circuit en parallèle est alimenté avec les valeurs d'entrée spécifiques à la personne, qui sont conduites à la seconde entrée du comparateur sous la forme de valeur de consigne, tandis que la différence des valeurs d'entrée du comparateur représente la grandeur de commande.

3. Dispositif selon la revendication 1 ou 2, caractérisé en ce qu'un convertisseur analogique/numérique est branché en amont du calculateur combiné au dispositif de commande, et un circuit d'interrogation d'un grand nombre de grandeurs d'entrée est branché en amont de ce dernier, et un convertisseur numérique/analogique est branché en aval et présente un circuit de sortie pour commander plusieurs circuits de commutation addressables.

4. Dispositif selon les revendications 1 à 3, caractérisé en ce que les lampes et/ou groupes de lampes sont réalisés de manière à être alimentés et commandés par un dispositif de commande commun combiné à un calculateur programmable, pour produire divers spectres combinés pendant la durée du rayonnement dans un domaine de longeurs d'ondes de l'ordre de 230 à 3000 nm.
